(19)

Europäisches Patentamt

European Patent Office

Office européen des brevets

(11) **EP 3 360 482 A1**

(12) **EUROPEAN PATENT APPLICATION**

(43) Date of publication:
15.08.2018 Bulletin 2018/33

(21) Application number: 17155385.2

(22) Date of filing: 09.02.2017

(51) Int Cl.:
A61B 6/00 (2006.01)     A61B 6/03 (2006.01)
A61B 6/04 (2006.01)     A61B 6/08 (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(71) Applicant: Koninklijke Philips N.V.
5656 AE Eindhoven (NL)

(72) Inventors:
• RUIJTERS, Daniël Simon Anna
  5656 AE Eindhoven (NL)
• VAN NIJNATTEN, Fred Simon Berend
  5656 AE Eindhoven (NL)
• OLIVAN BESCOS, Javier
  5656 AE Eindhoven (NL)

• HERMANS, Ronaldus Petrus Johannes
  5656 AE Eindhoven (NL)
• BASELMANS, Adrie
  5656 AE Eindhoven (NL)
• KLEIN-TEESELINK, Ina
  5656 AE Eindhoven (NL)
• SCHEEPENS, Jeroen Gerard
  5656 AE Eindhoven (NL)
• CARELS, Walter
  5656 AE Eindhoven (NL)
• GRUNHAGEN, Thijs
  5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) **ISO-CENTERING IN C-ARM COMPUTER TOMOGRAPHY**

(57) The present invention relates to iso-centering an object of interest prior to computer tomography (CT) scanning for examination of a patient in a C-arm CT examination apparatus. In order to provide facilitated positioning and to overcome or at least minimize these drawbacks, a C-arm CT examination apparatus (10) for CT scanning for examination of a patient is provided. The apparatus comprises a patient support unit, an acquisition unit, a data processing and control unit, and a display unit. The patient support unit is configured to bed a patient, free of motion, on the patient support. The acquisition unit is configured to acquire scout images of the patient including the object of interest in a respective position of the apparatus having position parameters. The data processing and control unit is configured to store the images and at least one respective position parameter including the iso-center position relative to the image; and to adjust the position of the apparatus roughly into a present position for CT scanning of the object of interest; and to select at least one iso-centering image from the stored images and, by geometrical calculation using, firstly, the at least one stored position parameter of the iso-centering image including the stored iso-center position and using, secondly, at least one position parameter of the present position of the apparatus including the present iso-center, adopting the appearance of the at least one iso-centering image, such that the appearance of the iso-centering image is according to the present position of the C-arm including the present iso-center. The display unit is configured to display the at least one adopted image on a display device together with and relative to a representation of the present iso-center for iso-centering.

EP 3 360 482 A1

Fig. 1

**Description**

FIELD OF THE INVENTION

The present invention relates to iso-centering an object of interest prior to computer tomography (CT) scanning for examination of a patient in a C-arm CT examination apparatus, and relates in particular to a method for applying geometry changes to pre-acquired scout images and.

BACKGROUND OF THE INVENTION

Cone beam computer tomography (CBCT) in the context of this invention involves a C-arm of an according CT examination apparatus following a circular rotational trajectory (or any other applicable trajectory that can be used to acquire tomographic reconstructions) around the object of interest, while acquiring (for example about 600) images. This allows to reconstruct, by a computer, a tomographic volume of the object of interest. The rotation point of this movement is known to be called the iso-center.

Before the rotational acquisition is made, the patient may be positioned on a table of the examination apparatus, such that the patient's anatomy of interest is within the field of view. This may be done by moving the patient table longitudinally and laterally (panning) and changing its height, while checking on the fluoroscopy image the relevant landmarks.

Iso-centering involves panning the table laterally and transversally, and adjusting the table height to put the relevant anatomy (the region of interest) in the iso-center position. To verify that the region of interest is indeed in the iso-center, the fluoroscopy pedal is kept pressed during the table movement, or immediately afterwards. Thereby the patient and the clinical staff are exposed to X-ray radiation.

Furthermore, to verify the position after table panning, it is desirable to look from the antero-posterior (AP) position, whereas to verify the position after table height changes, it is best to look from the lateral position. To do both on a monoplane C-arm, one needs to move the C-arm between AP and lateral positions, which takes time.

SUMMARY OF THE INVENTION

There may be a need to provide facilitated positioning and to overcome or at least minimize these drawbacks.

The object of the present invention is solved by the subject-matter of the independent claims; further embodiments are incorporated in the dependent claims.

It should be noted that the following described aspects of the invention apply also for the apparatus, the method, the computer program element and the computer readable medium.

According to a first aspect of the invention, a C-arm CT examination apparatus for CT scanning for examination of a patient is provided. The apparatus comprises a patient support unit, an acquisition unit, a data processing and control unit, and a display unit. The patient support unit is configured to bed a patient, free of motion, on the patient support. The acquisition unit is configured to acquire scout images of the patient including the object of interest in a respective position of the apparatus having position parameters. The data processing and control unit is configured to store the images and at least one respective position parameter including the iso-center position relative to the image; and to adjust the position of the apparatus roughly into a present position for CT scanning of the object of interest; and to select at least one iso-centering image from the stored images and, by geometrical calculation using, firstly, the at least one stored position parameter of the iso-centering image including the stored iso-center position and using, secondly, at least one position parameter of the present position of the apparatus including the present iso-center, adopting the appearance of the at least one iso-centering image, such that the appearance of the iso-centering image is according to the present position of the C-arm including the present iso-center. The display unit is configured to display the at least one adopted image on a display device together with and relative to a representation of the present iso-center for iso-centering.

According to an example, the apparatus is adopted to perform the method according to one of the following examples.

According to a second aspect of the invention, a method is provided for iso-centering an object of interest prior to CT scanning for examination of a patient in a C-arm CT examination apparatus, comprising the steps of:

- bedding a patient, free of motion, on a support, on a for example on a table, of a C-arm CT examination apparatus;
- acquiring an amount of (at least one) images (in 2D, so called scout scans) of the patient including the object of interest by an acquisition unit in the C-arm CT examination apparatus in a respective position of the apparatus (in particular, units of the apparatus like the acquisition unit and, in particular, its direction of view, CT scanning unit and, in particular, its direction of view, C-arm, L-arm, support and/or detector) having position parameters (in the three-dimensional space) and storing the images and at least one respective position parameter (preferably in 3D) including the iso-center position relative to the image. The scout images can be especially acquired for the purpose of iso-centering, or alternatively images that have been acquired during the procedure (e.g. diagnostic images or navigation images) can be re-used as scout images.;

- adjusting the position of the apparatus (if required, by driving its units) roughly (by view control of the user, e.g.) into a present position for CT scanning of the object of interest;
- selecting at least one iso-centering image from the stored images and, by geometrical calculation using, firstly, the at least one stored position parameter of the iso-centering image including the stored iso-center position and using, secondly, at least one position parameter of the present position of the apparatus including the present iso-center, adopting the appearance of the at least one iso-centering image, such that the appearance of the iso-centering image is according to the present position or orientation of the C-arm including the present iso-center (for example, such that the appearance of the iso-centering image is as if taken at the present C-arm orientation or, in another alternative, irrespective of the present C-arm orientation, while having an AP or lateral perspective, being only adopted such that the present iso-center is visible in the iso-centering image in true present proportions); and
- displaying the at least one adopted image on a display device together with and (preferably in correct proportions) relative to a representation of the present iso-center for iso-centering (by view control of the user, e.g.).

According to an example, the step of selecting an iso-centering image from the stored images is performed by selecting an image acquired in AP or lateral direction and thus having an AP or lateral perspective. Alternatively, the step of geometrical calculation is performed such that the adopted iso-centering image appears in an AP or lateral perspective. According to an example, particularly in one of these embodiments, the step of displaying the at least one adopted image on a display device is performed by displaying at least two iso-centering images next to each other on the display device including one having an AP perspective and one having a lateral perspective each together with and relative to a representation of the present iso-center.

According to a further exemplary embodiment, the step of storing the scout images is performed by storing parameters including the display range of the CT scan (preferably in correct proportions) relative to the image. Also, the step of geometrical calculation may be performed such that the appearance of the iso-centering image is according to the present position of the C-arm including the present range of the CT scan. And the step of displaying the adopted image on a display device may be performed by displaying (preferably in correct proportions) the present range of the CT scan.

According to a further exemplary embodiment, the step of selecting an iso-centering image from the stored images is performed by determining the iso-centering image as being the one, which comprises the object of interest most centrally and/or the position parameters of which are locally closest and/or least biased perspectively or least distorted relative to the parameters of the adjusted position of the apparatus.

According to a further exemplary embodiment, the step of acquiring scout images is performed by an X-ray or ultrasound or optical acquisition unit. Preferably, the step of acquiring scout images is performed by an acquisition unit attached to the C-arm and/or having a known orientation to the iso-center. Most preferably, the step of acquiring scout images is performed by the CT acquisition unit itself.

According to a further exemplary embodiment, (in order to apply any movements of the patient support or table onto the iso-centering image) the method comprises, at driving at least one unit of the apparatus (for example, the patient support) for iso-centering the object of interest (for example, by view control of the user, who watches the adopted image displayed together with and relative to a representation of the present iso-center on the display device), the subsequent step of repeating the step of adopting the appearance of the iso-centering image by repeating the geometrical calculation, now using, secondly, at least one position parameter of the now present position of the apparatus including the now present iso-center. In order to use a more appropriate stored image as the iso-centering image, the step of selecting the iso-centering image can be repeated and then repeating the step of adopting the appearance of the iso-centering image (in particular, by repeating the geometrical calculation now using, secondly, at least one position parameter of the now present position of the apparatus including the now present iso-center).

According to a further exemplary embodiment, the step of geometrical calculation is performed using at least one image parameter (for example, a point of origin within a coordinate system, size, scale, direction of view, vanishing point and/or perspective orientation) of the iso-centering image, the image parameter resulting from the respective position (i.e. also orientation) of the apparatus during acquisition of the iso-centering image and/or at least one image parameter of a CT scan, if taken in the present position (i.e. also orientation]) of the apparatus.

As scout images, fluoroscope and exposure acquisitions of the present exam of the present patient can be acquired and stored, along with their geometry information (C-arm viewing incidence, L-arm position, detector position, table height, etc.). During the iso-centering, the AP and lateral images that are closest to the current table position can be displayed. Furthermore, a graphic overlay can indicate the region of interest that will be reconstructed. They are translated and zoomed to match the current table position. This means that when a clinical user pans the table, the displayed images preferably pan along to reflect their content with respect to the current table position. Same applies for table height changes. When the table is moved to a position where a better matching image is available, the latter is displayed instead preferably.

Because of the perspective at image acquisition, it may be difficult to correct the complete visible scene in the image for the new patient support position. Therefore preferably, all transformations are calculated on the iso-center position that was stored with the respective image (which may be different from the current iso-center position) with respect to the source (and detector) of the acquisition unit.

The anatomical structures can be magnified differently due to the perspective and distance relative to the acquisition unit and/ or scanning unit (X-ray source and detector, e.g.). The magnification factor at a "fixed point" (certain height) above the table (e.g., 15 cm above the table) can be used as a compromise. In an example, it may be calculated as follows: m = SID / SOD, whereby m stands for magnification factor, SID for source-to-image distance (also called source-to-detector distance), and SOD for source-to-object distance (the distance of the X-ray source to the fixed point over the table). The SOD can be varied by changing the table height, while the SID can be varied by moving the detector.

Moving the table parallel to a recorded X-ray image, translates (pans) the image along with table movement (or translates a graphical representation indicating the reconstruction field of view by the same distance in the opposite direction).

When a table or support of a of a C-arm CT apparatus is moved along a vector aligned different than parallel with respect to the X-ray detector plane of a C-arm CT apparatus, the vector can be decomposed, according to an example, into a translational vector parallel to the detector plane, and a change of the SOD perpendicular to the detector plane.

During acquiring scout images, prior to the iso-centering, all images preferably are stored along with their geometry information (e.g., C-arm viewing incidence, L-arm position, detector position, table height, etc.) and time of creation. For example: A parameter set is denoted as P, which consists of a set of parameters $(p_1, ... , p_N)$, wherein $p_n$ stands for a particular parameter (e.g., SID, or table height, or creation time). A distance between two parameter sets then is defined as $d(P_1, P_2)$ and indicates the similarity between the two sets. The distance might for example be calculated as

$$d(P_1, P_2) = \sum_{n=1}^{N} w_n \cdot (p_{n,1} - p_{n,2}),$$

wherein $w_n$ indicates the contribution of each parameter difference. Other distance functions are also possible (e.g., setting the distance to infinite when a certain parameter $p_{n,1}$-$p_{n,2}$ is larger than a predefined threshold). Preferably, for each image that is acquired, the parameter set P, which reflects the geometry at the time of acquisition, is stored.

During iso-centering, it is preferred that an AP and lateral image are shown simultaneously (e.g., side by side). The AP and lateral image can be selected as follows: for each viewing direction (e.g., AP) a parameter set $P_{view}$ is assembled that sets the C-arm view parameters (e.g., rotation and angulation) to a predefined value (e.g. 0,0 for AP), and fills in the other parameters based on the current geometry position (table position, L-arm translation, etc.).

The parameter sets of all stored images are then compared to this $P_{view}$, using the earlier defined distance $d(P_{image}, P_{view})$ and the image with the smallest distance is selected for display.

As said, AP and lateral views preferably are presented simultaneously (e.g., side by side, or above each other, or on two separate monitors, etc.). Optionally, in the same view the typical reconstruction cube or cone (in particular showing the field of view of a C-arm CT) can be shown. During geometry changes (e.g. panning) of the patient support, the iso-centering images preferably are updated (panned or zoomed or replaced by an image with a smaller parameter distance) in real-time. Since feedback can be given to a user, like a physician using the apparatus or method according to one of the examples, regarding the effect of the current geometry state on the patient location with respect to the image, there is no need to radiate to create a new X-ray image for assessing the iso-centering and, possibly not even for assessing the field of view.

Small patient movements might be neglected, since iso-centering is typically not invalidated by a few millimeters offset. Large patient motions (e.g., repositioning the patient by clinical staff), however, can "invalidate" the stored images, as, in particular, the geometrical situation has changed and thus the stored position parameters do not reflect any more the present orientation of the object of interest depicted in the images. Automatic patient tracking (e.g., by depth cameras, or sensors connected to the patient) can be used to establish large patient movements. The user can also be given the option to manually clear the image buffer.

In the step of adopting, instead of moving and zooming the images with respect to a fixed reconstruction cube or square ore cone or trapeze, it is possible to move and zoom the reconstruction cube over a fixedly scaled image with the inverse quantity.

Instead of showing an AP and lateral view, any number of views from any predefined viewing angle can be shown. Instead of showing fixed views, the view may correspond to the current C-arm orientation. A view orientation may be defined by the user.

A threshold may be applied to the distance $d(P_{image}, P_{view})$, meaning that when the smallest found distance is larger than this threshold, no image is shown for that particular view. When no image is shown for a view, instruction regarding how to obtain this view may be provided instead.

Instead of showing the current isocenter and field of view on one or more previously acquired scout images, it is also possible to let the user indicate the desired isocenter and field of view on the images by user interaction (e.g. clicking on an image), and move the patient support (table) and/or C-arm (X-ray gantry) to the desired position by a motorized positioning system.

In order to reduce dose to sensitive organs, for example the eyes in a head scan, it is also possible to change the axis of rotation with respect to the orientation of the patient in addition to positioning the patient in x-, y-, and z-direction. Changing the axis of rotation of the X-ray system could for example be achieved by an angulated roll scan with the C-arm in side-position. Changing the orientation of the patient could for example be achieved by tilting the patient support (table).

As an example, the particular positioning can also be referred to as zero radiation iso-centering.

Instead of using previously acquired scout X-ray images, preferably taken by the CT scanning unit (in particular, X-ray source and detector), also images of different sources can be used. For example, if the region of interest is already visualized by e.g. ultra sound, and the ultra sound is registered geometrically with respect to the C-arm CT apparatus, then these ultra sound images can be used as well. Or optical images can be used, e.g., acquired by cameras attached to the C-arm gantry.

Instead of using the examples of the method on a Cone Beam CT, the examples can also be applied for any other X-ray acquisition for which defining a region of interest is relevant.

These and other aspects of the present invention will become apparent from and be elucidated with reference to the embodiments described hereinafter.

BRIEF DESCRIPTION OF THE DRAWINGS

Exemplary embodiments of the invention will be described in the following with reference to the following drawings:

Fig. 1 is a schematic side view of an example of a C-arm CT examination apparatus;
Fig. 2a, 2b are schematic views of an example of a C-arm CT examination apparatus in AP and lateral view position of the C-arm during examination of a patient;
Fig. 3 is a flow diagram of an example of a method; and
Fig. 4 is an example of a display of a display unit during examination of a patient.

DETAILED DESCRIPTION OF EMBODIMENTS

In the following, the invention is exemplarily described as being used in the context of a C-arm CT examination apparatus 10.

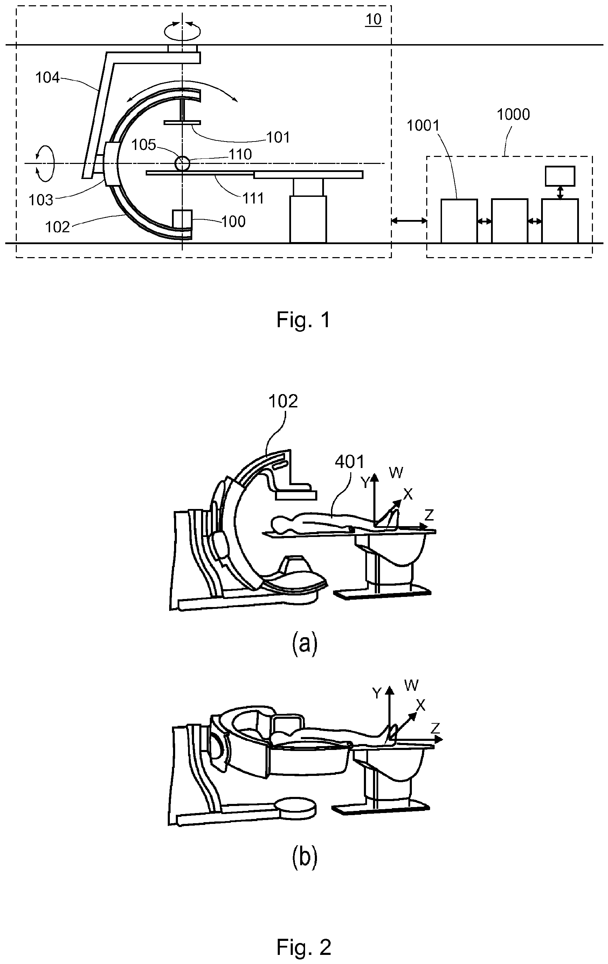

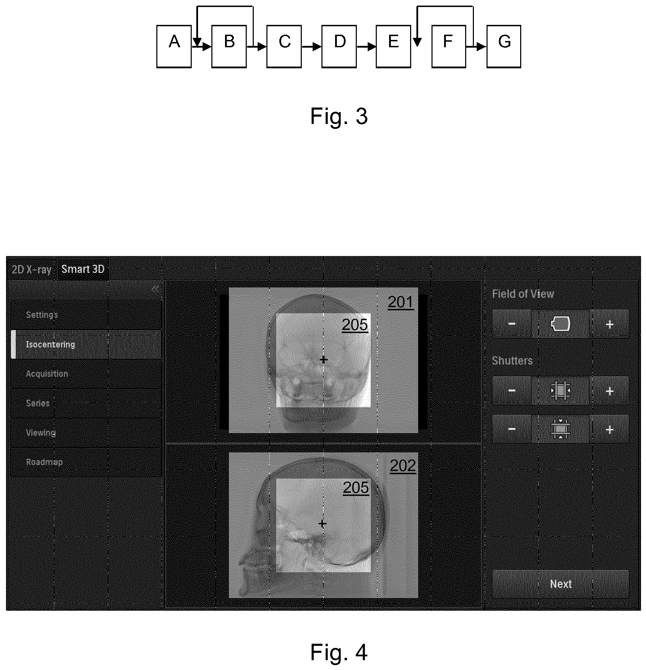

Figs. 1 and 2 schematically show a C-arm CT examination apparatus 10 for CT scanning for examination of a patient 401, adopted to perform the method according to Fig. 3.

According to Fig. 3, a flow diagram represents the following steps of a method for iso-centering an object of interest 110.

Iso-centering means adjusting geometrical, spatial parameters relative to each other of the units of the apparatus 10, namely the L-arm 104, C-arm 102, X-ray source 100, X-ray detector 101 and patient support 111, such that position of the iso-center 105 of the apparatus 10-i.e. intersection 105 of the horizontal rotation axis (depicted in chain-line) of the C-arm 102 and of the view axis (direction of view of the CT scanning and image acquisition unit 100, 101; depicted in chain-line, vertical in the depicted state of the apparatus 10) is positioned within the object of interest 100, e.g., the head 110 of the patient 401) prior to computer tomography (CT) scanning for examination of a patient 401 in a C-arm CT examination apparatus 10.

As indicated in Fig. 1, in an example, the C-arm CT examination apparatus 10 comprises (not shown in detail) a patient support unit, an acquisition unit, a data processing and control unit and a display unit. The patient support unit is configured to bed a patient, free of motion, on the patient support. The acquisition unit is configured to acquire scout images of the patient including the object of interest in a respective position of the apparatus having position parameters. The data processing and control unit is configured to store the images and at least one respective position parameter including the iso-center position relative to the image; and to adjust the position of the apparatus roughly into a present position for CT scanning of the object of interest; and to select at least one iso-centering image from the stored images and, by geometrical calculation using, firstly, the at least one stored position parameter of the iso-centering image including the stored iso-center position and using, secondly, at least one position parameter of the present position of the apparatus including the present iso-center, adopting the appearance of the at least one iso-centering image, such that the appearance of the iso-centering image is according to the present position of the C-arm including the present iso-center. The display unit is configured to display the at least one adopted image on a display device together with and relative to a representation of the present iso-center for iso-centering.

The method comprises:

A) bedding a patient 401, free of motion, on a support 111 of a C-arm CT examination apparatus 10;
B) acquiring scout images of the patient 401 including the object of interest 110 by an X-ray acquisition unit 100, 101, namely the CT acquisition unit (the CT scanning unit 100, 101 itself) in the C-arm CT examination apparatus 10 in a respective position of the apparatus having position parameters and storing the images and at least one respective position parameter including the iso-center position relative to the image and including the display range of the CT scan 205 relative to the image in a data storage 1001 of a processing unit 1000;

C) adjusting the position of the apparatus 10 roughly into a present position for CT scanning of the object of interest 110;

D) selecting, from the stored images, one iso-centering image acquired in AP direction (Fig. 2a) and one iso-centering image acquired in lateral direction (Fig. 2b) by determining the respective iso-centering image as being the one, which comprises the object of interest 110 most centrally and complete and respectively, by geometrical calculation using, firstly, the at least one stored position parameter of the respective iso-centering image including the stored iso-center position and using, secondly, at least one position parameter of the present position of the apparatus 10 including the present iso-center 105, adopting the appearance of the two iso-centering images, such that the appearance of the iso-centering images is according to the present position of the C-arm 102 including the present iso-center 105 and including the present range of the CT scan 205;

E) displaying the two adopted iso-centering images next to each other on a display device, one image 201 having the AP perspective and one image 202 having the lateral perspective, each image displayed together with and relative to a representation of the present iso-center (+) and the present range of the CT scan 205 (see also Fig. 4).

In an option it is provided;

F) at driving at least one unit 100-104 of the apparatus 10 for iso-centering the object of interest 110, repeating the step of selecting the iso-centering image by determining, if the previously selected respective iso-centering image still (after the driving) is the one, which comprises the object of interest 110 most centrally and complete, and then of adopting the appearance of the iso-centering image by repeating the geometrical calculation, now using, secondly, at least one position parameter of the now present position of the apparatus including the now present iso-center; and

G) after iso-centering, CT scanning the region of interest 110 using the scanning unit 100, 101.

Fig. 4 shows an example of a display of a display unit during examination of a patient. The display is provided also as an interface for the user, e.g. to actively control the displayed content as indicated with "-" and "+" buttons.

Moreover, although in the previous description the word "device" has been used, whenever the word "device" is mentioned it is understood that this might indeed refer to a full device but also to just a fraction of a device.

Moreover, although in the previous description the word "apparatus" has been used, whenever the word "apparatus" is mentioned it is understood that this might indeed refer to a full apparatus but also to just a fraction of an apparatus.

It is understood that, without repeating here all the examples and explanations provided with reference to the method of the invention, the apparatus of the invention is intended as being arranged to carry out the above described method steps. Thus, all of the above examples and explanations, although provided with reference to the method, are also to be intended as being implemented by the apparatus. This can be achieved, for example, by suitable hardware and/or software.

It is understood that, without repeating here all the explanations, examples, features and/or advantages provided with reference to the apparatus, the method of the invention is intended to be configured to carry out the method steps for which the apparatus is configured to. Thus, all the above examples, explanations, features and/or advantages, although provided with reference to the apparatus, are also to be intended as being provided in an analogous manner for the method.

In another exemplary embodiment of the present invention, a computer program or a computer program element is provided that is characterized by being adapted to execute the method steps of the method according to one of the preceding embodiments, on an appropriate system.

The computer program element might be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items recited in the claims. The mere fact that certain measures are re-cited in mutually different dependent claims does not indicate that a combination of these measures cannot be used to advantage. Any reference signs in the claims should not be construed as limiting the scope.

**Claims**

1. A C-arm CT examination apparatus (10) for CT scanning for examination of a patient, the apparatus comprising:

   - a patient support unit;
   - an acquisition unit;
   - a data processing and control unit; and
   - a display unit;

   wherein the patient support unit is configured to bed a patient, free of motion, on the patient support; wherein the acquisition unit is configured to acquire scout images of the patient including the object of interest in a respective position of the apparatus having position parameters;
   wherein the data processing and control unit is configured to store the images and at least one respective position parameter including the iso-center position relative to the image; and to adjust the position of the apparatus roughly into a present position for CT scanning of the object of interest; and to select at least one iso-centering image from the stored images and, by geometrical calculation using, firstly, the at least one stored position parameter of the iso-centering image including the stored iso-center position and using, secondly, at least one position parameter of the present position of the apparatus including the present iso-center, adopting the appearance of the at least one iso-centering image, such that the appearance of the iso-centering image is according to the present position of the C-arm including the present iso-center;
   wherein the display unit is configured to display the at least one adopted image on a display device together with and relative to a representation of the present iso-center for iso-centering.

2. A method for iso-centering an object of interest prior to CT scanning for examination of a patient in a C-arm CT examination apparatus, comprising the steps of:

   - bedding (A) a patient, free of motion, on a support of a C-arm CT examination apparatus;
   - acquiring (B) scout images of the patient including the object of interest by an acquisition unit in the C-arm CT examination apparatus in a respective position of the apparatus having position parameters and storing the images and at least one respective position parameter including the iso-center position relative to the image;
   - adjusting (C) the position of the apparatus roughly into a present position for CT scanning of the object of interest;
   - selecting (D) at least one iso-centering image from the stored images and, by geometrical calculation using, firstly, the at least one stored position parameter of the iso-centering image including the stored iso-center position and using, secondly, at least one position parameter of the present position of the apparatus including the present iso-center, adopting the appearance of the at least one iso-centering image, such that the appearance of the iso-centering image is according to the present position of the C-arm including the present iso-center; and
   - displaying (E) the at least one adopted image on a display device together with and relative to a representation of the present iso-center for iso-centering.

3. Method according to claim 2, wherein the step of selecting an iso-centering image from the stored images is performed by selecting an image acquired in AP or lateral direction and thus having an AP or lateral perspective.

4. Method according to claim 2 or 3, wherein the step of geometrical calculation is performed such that the adopted iso-centering image appears in an AP or lateral perspective.

5. Method according to one of claims 2 to 4, wherein the step of displaying the at least one adopted image on a display device is performed by displaying at least two iso-centering images next to each other on the display device including one having an AP perspective and one having a lateral perspective each together with and relative to a representation of the present iso-center.

6. Method according to one of claims 2 to 5, wherein the step of storing the scout images is performed by storing parameters including the display range of the CT scan relative to the image; and/or wherein the step of geometrical calculation is performed such that the appearance of the iso-centering image is according to the present position of the C-arm including the present range of the CT scan; and/or wherein the step of displaying the adopted image on a display device is performed by displaying the present range of the CT scan.

7. Method according to one of claims 2 to 6, wherein the step of selecting an iso-centering image from the stored images is performed by determining the iso-centering image as being the one, which comprises the object of interest most centrally and/or the position parameters of which are locally closest and/or least biased perspectively relative to the parameters of the adjusted position of the apparatus.

8. Method according to one of claims 2 to 7, wherein the step of acquiring scout images is performed by an X-ray or ultrasound or optical acquisition unit.

9. Method according to one of claims 2 to 8, wherein the step of acquiring scout images is performed by an acquisition unit attached to the C-arm and/or having a known orientation to the iso-center.

10. Method according to one of claims 2 to 9, wherein the step of acquiring scout images is performed by the CT acquisition unit.

11. Method according to one of claims 2 to 10, comprising, at driving at least one unit of the apparatus for iso-centering the object of interest, the subsequent step of:

   - repeating the step of adopting the appearance of the iso-centering image by repeating the geometrical calculation, now using, secondly, at least one position parameter of the now present position of the apparatus including the now present iso-center; or
   - repeating the step of selecting the iso-centering image and then adopting the appearance of the iso-centering image by repeating the geometrical calculation, now using, secondly, at least one position parameter of the now present position of the apparatus including the now present iso-center.

12. Method according to one of claims 2 to 11, wherein the step of geometrical calculation is performed using at least one image parameter of the iso-centering image, the image parameter resulting from the respective position of the apparatus during acquisition of the iso-centering image and/or at least one image parameter of a CT scan, if taken in the present position of the apparatus.

13. A computer program element for controlling an apparatus according to claim 1, which, when being executed by a processing unit, is adapted to perform the method steps of one of the claims 2 to 12.

14. A computer readable medium having stored the program element of claim 13.

Fig. 1

(a)

(b)

Fig. 2

Fig. 3

Fig. 4

Europäisches
Patentamt

European
Patent Office

Office européen
des brevets

# EUROPEAN SEARCH REPORT

Application Number

EP 17 15 5385

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 926 434 B1 (KONINKL PHILIPS NV [NL]) 8 April 2015 (2015-04-08) * (192); paragraphs [0021], [0022] * | 1-14 | INV. A61B6/00 A61B6/03 A61B6/04 A61B6/08 |
| A | WO 2015/121301 A1 (SIEMENS AG [DE]) 20 August 2015 (2015-08-20) * (308) * | 1-14 | |
| A | US 2015/094567 A1 (PFISTER MARCUS [DE]) 2 April 2015 (2015-04-02) * paragraph [0111] * | 1-14 | |

TECHNICAL FIELDS
SEARCHED      (IPC)

A61B

The present search report has been drawn up for all claims

| Place of search | Date of completion of the search | Examiner |
|---|---|---|
| Munich | 7 November 2017 | Anscombe, Marcel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 15 5385

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2017

| Patent document cited in search report | | Publication date | Patent family member(s) | | Publication date |
|---|---|---|---|---|---|
| EP 1926434 | B1 | 08-04-2015 | CA | 2622227 A1 | 22-03-2007 |
| | | | CN | 101262820 A | 10-09-2008 |
| | | | EP | 1926434 A2 | 04-06-2008 |
| | | | JP | 5203206 B2 | 05-06-2013 |
| | | | JP | 2009507600 A | 26-02-2009 |
| | | | KR | 20080051137 A | 10-06-2008 |
| | | | US | 2008198972 A1 | 21-08-2008 |
| | | | WO | 2007031945 A2 | 22-03-2007 |
| WO 2015121301 | A1 | 20-08-2015 | NONE | | |
| US 2015094567 | A1 | 02-04-2015 | DE | 102013219737 A1 | 02-04-2015 |
| | | | US | 2015094567 A1 | 02-04-2015 |

EPO FORM P0459

For more details about this annex : see Official Journal of the European Patent Office, No. 12/82